**Europäisches Patentamt**

**European Patent Office**

**Office européen des brevets**

(11) Publication number: **0 268 968**
**B1**

(12) **EUROPEAN PATENT SPECIFICATION**

(45) Date of publication of patent specification: **06.02.91**

(21) Application number: **87116888.6**

(22) Date of filing: **16.11.87**

(51) Int. Cl.⁵: **A 23 L 3/28,** A 23 L 2/24, A 61 L 9/20

(54) Fluid sterilizing process and device.

(30) Priority: **21.11.86 IT 6786386**

(43) Date of publication of application:
**01.06.88 Bulletin 88/22**

(45) Publication of the grant of the patent:
**06.02.91 Bulletin 91/06**

(84) Designated Contracting States:
**CH DE ES FR GB GR LI NL SE**

(56) References cited:
**DE-C- 885 344**
**US-A-2 586 625**
**US-A-4 534 282**

(73) Proprietor: **ULTRAVIOLET TECHNOLOGY ITALIA S.r.l.**
**Corso Felice Cavallotti, 26**
**I-28100 Novara (IT)**

(72) Inventor: **Striglia Silvio**
**Via Aronco 28**
**28053 Castelletto Ticino (IT)**

(74) Representative: **Prato, Roberto et al**
**STUDIO TORTA Società Semplice Via Viotti 9**
**I-10121 Torino (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

The present invention relates to a process for sterilizing fluids.

The effective sterilizing action of ultraviolet rays on fluids penetrated by the same is by now a well established fact.

As yet, however, this property of ultraviolet rays has generally been employed for sterilizing water or other alimentary fluids. In particular, devices are known for purifying and sterilizing drinks, such as water or similar fluids transparent to ultraviolet rays, on which the drink is fed through at least one vessel or tubular duct. The said duct is defined by a wall at least partly transparent to ultraviolet rays, and is set up in front of at least one source of the same. The ultraviolet rays produced by the said source thus pass through the said transparent wall and the drink, which is also transparent, so as to enable in-depth bombardment and effective sterilization of the drink particles.

A major drawback of known purifying-sterilizing devices of the aforementioned type is that they are limited to drinks transparent to ultraviolet rays, and cannot be employed for fluids, such as syrups, lubricating oils or emulsions, that are impervious to ultraviolet rays and, therefore, usually require a much more expensive sterilizing process.

The aim of the present invention is to provide a fluid sterilizing process designed to overcome the aforementioned drawback.

With this aim in view, according to the present invention, there is provided a fluid sterilizing process, characterised by the fact that it comprises stages consisting in:

— feeding the particles of a fluid, even one impervious to ultraviolet rays, along a narrow passage defined by two walls, at least one of which is formed from material transparent to ultraviolet rays, and at least one of which is formed from unwettable material designed to produce, in the fluid flowing along the said passage, microvortices substantially equal in diameter to the width of the said passage;

— superficially bombarding the said particles with ultraviolet rays, as the said particles traveling along the said passage swirl into substantial contact with the said transparent wall; the said ultraviolet rays being produced by at least one source located outside the said passage and in front of the said transparent wall.

According to the above process, sterilization is achieved, not by the said ultraviolet rays penetrating the fluid, but by means of superficial bombardment, thus substantially overcoming the functional limitation posed by non-transparent fluids.

According to the present invention, there is also provided a fluid sterilizing device, even for fluids impervious to ultraviolet rays, characterised by the fact that it comprises duct means for a stream of unsterilized fluid particles, said duct means, in turn, comprising two opposite walls defining a relatively narrow passage, at least one of the said walls being formed, at least partially, from material transparent to ultraviolet rays, and at least one of the said walls being formed from unwettable material; and at least one source of ultraviolet rays, located facing the said transparent wall of the said passage; the width of the said passage being approximately the same size as the diameter of vortices induced in the said stream, in use, by the said unwettable wall.

An embodiment of the present invention will be described by way of example with reference to the accompanying drawings, in which:

Fig. 1 shows a section of a preferred embodiment of a fluid sterilizing device in accordance with the teachings of the present invention;

Fig. 2 shows a section along line II—II in Fig. 1.

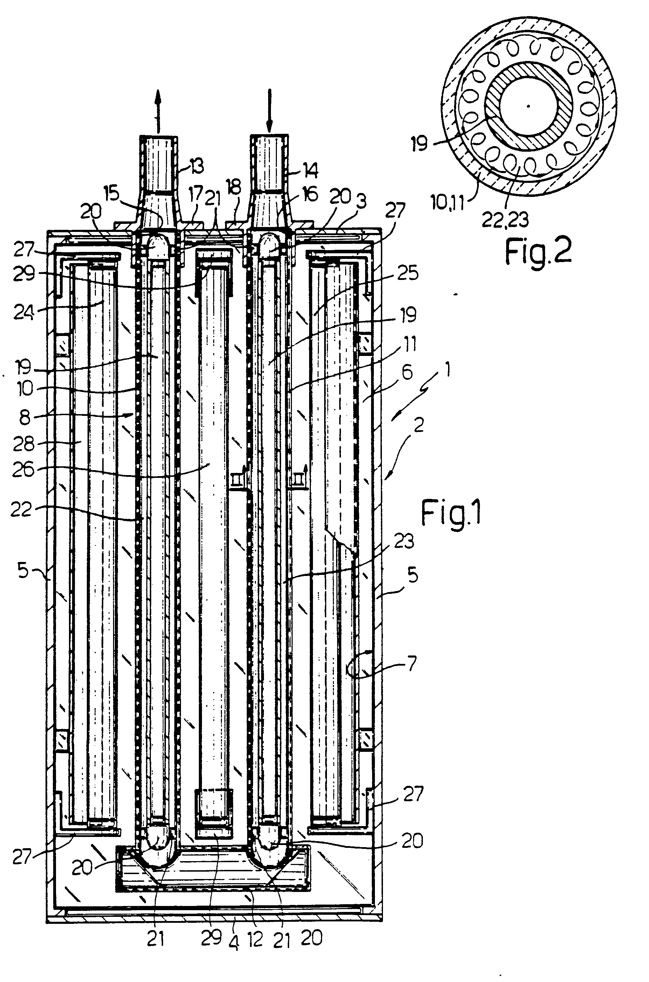

Number 1 in Fig. 1 indicates a fluid purifying-sterilizing device, even for fluids impervious to ultraviolet rays, comprising an outer casing 2 preferably formed from metal and in the form of a rectangular parallelepipedon.

The said casing 2 comprises a top wall 3, a bottom wall 4, a first pair of parallel side walls 5, and a second pair of parallel side walls 6 (only one of which is shown) perpendicular to walls 5.

Inside a chamber 7 defined by the said walls 3, 4, 5 and 6, there is housed a duct 8 for the said unsterilized fluid, which duct 8 may be of any shape, but, in the example shown, is substantially U-shaped with its concave side facing upwards. In more detail, the said duct 8 comprises a downward duct 10 and an upward duct 11 parallel with each other, formed from unwettable material transparent to ultraviolet rays, and connected at their respective bottom ends by a horizontal duct 12. The top ends of ducts 10 and 11 are engaged inside respective tubular ends of an outlet pipe 13 and inlet pipe 14 respectively, which pipes 13 and 14 extend through respective holes 15 and 16 formed through wall 3, and are connected to the said wall 3 via respective flanges 17 and 18.

Each of ducts 10 and 11 houses a tubular element 19 preferably formed from metal and closed at its opposite ends by plugs 20, each of which is secured to the inner surface of respective duct 10 or 11 by means of radial fins 21.

The said tubular elements 19 extend along substantially the entire length of respective ducts 10 and 11 and define, with the same, respective toroidal passages 22 and 23 along which the unsterilized fluid flows.

In the example shown, three fluorescent infrared lamps, 24, 25, 26, are mounted inside the said chamber 7. Lamps 24 and 25 are connected by means of respective end brackets 27 to respective walls 5, in a position adjacent to ducts 10 and 11 respectively, and present respective reflecting paraboloids 28 for concentrating the emitted rays on to ducts 10 and 11. Lamp 26, on the other hand, is located midway between ducts 10 and 11, and is supported by end brackets 29 connected to one of walls 6.

In actual use, the unsterilized fluid is fed into inlet pipe 14 by a supply network (not shown) and flows along duct 8 by successively flowing along passage 23, duct 12 and passage 22, as far as outlet pipe 13.

As it flows along toroidal passages 22 and 23, the unsterilized fluid assumes a swirling motion due to the fact that, as ducts 10 and 11 are formed from unwettable material, such as TEFLON®, the fluid particles substantially contacting the inner surfaces of ducts 10 and 11 encounter substantially no frictional resistance and, therefore, tend to travel faster than the other particles. The size of the vortices formed inside passages 22 and 23 remains substantially constant for a given flow rate. Consequently, by selecting, for passages 22 and 23, a width approximately equal to the diameter of the vortices produced at the required flow rate, all the unsterilized fluid particles may be brought successively into contact with the inner surfaces of ducts 10 and 11, that is, into a position enabling the particles to be sterilized by a superficial bombardment of ultraviolet rays emitted by lamps 24, 25 and 26.

For example, according to a first variation (not shown), duct 8 may consist of a coil, or be of any appropriate shape other than the one described herein.

Similarly, according to a further variation (not shown), passages 22 and 23 may be flat, instead of toroidal as in the example shown.

According to a further variation (not shown), tubular elements 19 may be formed from material designed to reflect ultraviolet rays, or, they too, from unwettable material transparent to ultraviolet rays.

According to a further variation (not shown), the ultraviolet lamps employed may differ in number and/or be positioned differently from those described herein.

Finally, according to a further variation (not shown), device 1 as described herein may constitute only one sterilizing unit on a system comprising a number of such units connected in various ways and housed either in respective casings 2 or in a single common casing.

## Claims

1. A fluid sterilizing process, characterised by the fact that it comprises stages consisting in:
— feeding the particles of a fluid, even one impervious to ultraviolet rays, along a narrow passage defined by two walls, at least one of which is formed from material transparent to ultraviolet rays, and at least one of which is formed from unwettable material designed to produce, in the fluid flowing along the said passage, microvortices substantially equal in diameter to the width of the said passage;
— superficially bombarding the said particles with ultraviolet rays, as the said particles traveling along the said passage swirl into substantial contact with the said transparent wall; the said ultraviolet rays being produced by at least one source located outside the said passage and in front of the said transparent wall.

2. A fluid sterilizing device, even for fluids impervious to ultraviolet rays, characterised by the fact that it comprises duct means for a stream of unsterilized fluid particles, said duct means, in turn, comprising two opposite walls defining a relatively narrow passage, at least one of the said walls being formed, at least partially, from material transparent to ultraviolet rays, and at least one of the said walls being formed from unwettable material; and at least one source of ultraviolet rays, located facing the said transparent wall of the said passage; the width of the said passage being approximately the same size as the diameter of vortices induced in the said stream, in use, by the said unwettable wall.

3. A device as claimed in Claim 2, characterised by the fact that the said passage is toroidal in shape; the said two walls being tubular and coaxial with each other.

4. A device as claimed in Claim 3, characterised by the fact that a first outer wall of the said tubular walls is formed from the said unwettable material transparent to ultraviolet rays; the said source of ultraviolet rays being located outside the said outer wall.

## Patentansprüche

1. Verfahren zur Sterilisation von Flüssigkeiten, gekennzeichnet durch Verfahrensschritte, die darin bestehen,
— das die Teilchen einer Flüssigkeit, auch einer für ultraviolette Strahlung undurchsichtigen, durch einen engen Durchgang geführt werden, der durch zwei Wände definiert ist, von denen die eine aus einem für ultraviolette Strahlung durchsichtigen Material und mindestens eine von beiden aus einem unbenetzbaren Material gebildet ist, ausgestaltet, um in der Flüssigkeit, die durch diesen Durchgang fliesst, Mikrowirbel im wesentlichen mit demselben Durchmesser wie die Breite dieses Durchgangs zu verursachen, und
— dass diese Teilchen an der Oberfläche mit einer ultravioletten Strahlung bombardiert werden, wenn beim Wirbeln diese Teilchen, die durch diesen Durchgang vorwärtsschreiten, im wesentlichen in Kontakt mit dieser durchsichtigen Wand kommen, wobei die ultraviolette Strahlung durch mindestens eine Quelle erzeugt wird, die sich ausserhalb dieses Durchgangs und gegenüber dieser durchsichtigen Wand befindet.

2. Vorrichtung zur Sterilisation von Flüssigkeiten, auch einer für ultraviolette Strahlung undurchsichtigen, dadurch gekennzeichnet, dass sie Führungsmittel für einen Strom von nichtsterilisierten Flüssigkeitsteilchen umfasst, wobei diese Führungsmittel ihrerseits zwei gegenüberliegende Wände aufweisen, die einen relativ engen Durchgang definieren, und wobei mindestens eine dieser Wände wenigstens teilweise aus einem für ultraviolette Strahlung durchsichtigen Material und mindestens eine dieser Wände aus einem nichtbenetzbaren Material gebildet ist, und dass sich wenigstens eine Ultraviolett-Strahlungsquelle gegenüber dieser durchsichtigen Wand dieses Durchgangs befindet, wobei die Breite dieses Durchgangs ungefähr gleich wie der Durchmesser der Wirbel ist, die in diesem Strom, in Gebrauch, durch diese nichtbenetzbare Wand induziert werden.

3. Vorrichtung nach Anspruch 2, dadurch gekennzeichnet, dass dieser Durchgang eine toroidale Form aufweist, wobei diese zwei Wände tubular und gegeneinander koaxial angeordnet sind.

4. Vorrichtung nach Anspruch 3, dadurch gekennzeichnet, dass von diesen rohrförmigen Wänden eine erste Aussenwand aus diesem nichtbenetzbaren, für ultraviolette Strahlung durchsichtigen Material gebildet ist, wobei die Ultraviolett-Strahlungsquelle aussenseitig bezüglich dieser Aussenwand angeordnet ist.

## Revendications

1. Un procédé de stérilisation de fluide, caractérisé en ce qu'il comporte les étapes comprenant:

— l'alimentation de particules d'un fluide, y compris d'un fluide imperméable, aux rayons ultraviolets, dans un passage étroit délimité par deux parois, dont au moins l'une d'elles est formée à partir d'un matériau perméable aux rayons ultraviolets et dont au moins l'une d'elles est formée à partir d'un matériau non-mouillable, conçu pour produire dans le fluide s'écoulant dans ledit passage des micro-tourbillons sensiblement d'un diamètre égal à la largeur dudit passage,

— le bombardement en surface aux rayons ultraviolets desdites particules pendant l'écoulement desdites particules le long dudit passage en tourbillonnant, sensiblement au contact de ladite paroi perméable, lesdits rayons ultraviolets étant émis par au moins une source située à l'extérieur dudit passage et en face de ladite paroi perméable.

2. Un dispositif de stérilisation de fluide, y compris de fluides imperméables aux rayons ultraviolets, caractérisé en ce qu'il comprend des moyens formant conduit pour la circulation d'un courant de particules de fluide non-stérilisées, lesdits moyens formant conduit comprenant, de leur côté, deux parois opposées délimitant un passage relativement étroit, au moins l'une desdites parois étant formée, au moins en partie, à partir d'un matériau perméable aux rayons ultraviolets et au moins l'une desdites parois étant formée à partir d'un matériau non-mouillable, et au moins une source de rayons ultraviolets disposée en face de ladite paroi perméable dudit passage, la largeur dudit passage étant approximativement de même dimension que le diamètre des tourbillons formés en service dans ledit courant par ladite paroi non-mouillable.

3. Un dispositif selon la revendication 2, caractérisé en ce que ledit passage est de forme toroïdale, lesdites deux parois étant tubulaires et coaxiales l'une par rapport à l'autre.

4. Un dispositif selon la revendication 3, caractérisé en ce que la première paroi extérieure desdites parois tubulaires est formée à partir dudit matériau non-mouillable, perméable aux rayons ultraviolets, ladite source de rayons ultraviolets étant située à l'extérieur de ladite paroi extérieure.

Fig.2

Fig.1